(19) Europäisches Patentamt

European Patent Office

Office européen des brevets

(11) Veröffentlichungsnummer: **0 360 029 B1**

(12) **EUROPÄISCHE PATENTSCHRIFT**

(45) Veröffentlichungstag der Patentschrift: **04.08.93**

(51) Int. Cl.5: **C12Q 1/533**, C12Q 1/37

(21) Anmeldenummer: **89115759.6**

(22) Anmeldetag: **26.08.89**

(54) **Verfahren und Assay zur Konzentrationsbestimmung von Cyclosporinen.**

(30) Priorität: **07.09.88 DD 319577**

(43) Veröffentlichungstag der Anmeldung:
**28.03.90 Patentblatt 90/13**

(45) Bekanntmachung des Hinweises auf die
Patenterteilung:
**04.08.93 Patentblatt 93/31**

(84) Benannte Vertragsstaaten:
**AT BE CH DE ES FR GB IT LI LU NL SE**

(56) Entgegenhaltungen:

NATURE, Band 337, 2. Februar 1989, Seiten
476-478; G. FISCHER et al.: "Cyclophilin and
peptidyl-prolyl cis-trans isomerase are pobably identical proteins"

NATURE, Band 337, 2. Februar 1989, Seiten
473-475; N. TAKAHASHI et al.:
"Peptidyl-prolyl cis-trans isomerase is the
cyclosporin A-binding protein cyclophilin"

(73) Patentinhaber: **ARZNEIMITTELWERK DRESDEN
GmbH
Postfach 01 01 31/01 01 32
O-8122 Radebeul(DE)**

(72) Erfinder: **Fischer, Gunter, Dr.
Otto Kanning-Str. 11
O-4059 Halle(DE)**
Erfinder: **Küllertz, Gerhard, Dr.
Leninallee 18
O-1800 Brandenburg(DE)**

(74) Vertreter: **Puchberger, Rolf, Dipl. Ing. et al
Patentanwälte, Dipl. Ing. Georg Puchberger
Dipl. Ing. Rolf Puchberger Dipl. Ing. Peter
Puchberger Singerstrasse 13 Postfach 55
A-1010 Wien (AT)**

THE JOURNAL OF BIOLOGICAL CHEMISTRY, Band 262, Nr. 35, 15. December 1987, Seiten 17144-17148, The American Society for Biochemistry and Molecular Biology Inc., US; H.P. BÄCHINGER: "The influence of peptidyl-prolyl cis-trans isomerase on the in Vitro folding of type III collagen"

CHEMICAL ABSTRACTS, Band 102, Nr. 13, 1. April 1985, Seite 288, Zusammenfassung Nr. 108562y, Columbus, Ohio, US; G. FISCHER et al.: "Detection of enyme catalysis for cis-trans-isomerization of peptide bonds using proline-containing peptides", & BIOMED. BIOCHIM. ACTA 1984, 43(10), 1101-11

CHEMICAL ABSTRACTS, Band 92, Nr. 3, 21. Januar 1980, Seite 269, Zusammenfassung Nr. 17698f, Columbus, Ohio, US; M.J CASTILLO et al.: "Sensitive substrates for human leukocyte and porcine pancreatic elastase: a study of the merits of various chromophoric and fluorgenic leaving groups in assays for serine proteases", & ANAL. BIOCHEM. 1979, 99(1), 53-64

**Beschreibung**

Die Erfindung betrifft ein Verfahren zur Konzentrationsbestimmung von Cyclosporin und seinen biologisch wirksamen Derivaten. Es kann in medizinischen und biologischen Laboratorien genutzt werden. Die Bestimmung hat bei Patienten mit Organtransplantaten und Autoimmunerkrankungen eine diagnostische Bedeutung.

Der Nachweis von Cyclosporinkonzentration ist wichtig zur Therapieüberwachung bei Patienten, die mit Medikamenten therapiert werden, die die Substanzklasse der Cyclosporine als therapeutisch wichtiges Pharmakon besitzen.

Die Konzentration von Cyclosporin und seinen biologisch wirksamen Derivaten ist in wäßrigen Systemen, vor allem in biologischen Flussigkeiten, insbesondere im Blutserum oder Plasma, aber auch in Liquor, Synovialflüssigkeit, Urin oder suspensionen von Blutzellen bzw. Gewebehomogenaten zu bestimmen.

Hauptsächlich wird die Bestimmung der Konzentration von Cyclosporin und seinen Derivaten bei der Einstellung und Überwachung des therapeutisch sinnvollen Konzentrationsbereiches in der Humanmedizin bei Patienten mit transplantierten Organen angewendet, die zur Vermeidung von Abstoßungsreaktionen Verbindungen, wie Cyclosporin, als Medikament erhalten.

Aufgrund der relativ geringen therapeutisch sinnvollen Breite der Medikamentation ist es zur Vermeidung von immunologischen Reaktionen gegenüber dem Transplantat bei zu geringer Dosierung bzw. zur Vermeidung von toxischen Nebenwirkungen bei zuhoher Dosierung notwendig, die Konzentration des Pharmakons in Körperflüssigkeiten von Patienten zu messen. Die Notwendigkeit dieser Konzentrationsbestimmung ergibt sich weiterhin aus den inter-individuellen Unterschieden der natürlichen Elimination dieser Pharmaka und aus der sich bei längerer Medikamentation ändernden intraindividuellen Eliminationsrate, die für eine differenzierte Dosierung des Medikamentes Voraussetzung ist.

Üblicherweise erfolgt die Konzentrationsbestimmung von Cyclosporin und seinen Derivaten mittels immunologischer Verfahren (Keown P.A., Stiller C.R.Sinclair N.R. et al. The clinical relevance of cyclosporine blood levels as measured by radioimunoassay, Transplant Proc. (1983), 15, 2438-2441) oder chromatografischer Verfahren (Kabra P.K. Wall J.H. Dimson P. Automated solid -phase extraction and liquid chromatography for assay of cyclosporine in whole blood. Clinical chemistry (1987), 33, 2272-2274).

Immunologische Verfahren stützen sich auf die Herstellung geeigneter Antikörper gegen das interessierende Cyclosporin. Mit Hilfe dieses Antikörpers gelingt es dann, mittels der aufwendigen Methoden des Inmunoassays die Konzentration des Antigens zu ermitteln. Die Nachteile dieser Methode sind insbesondere die manuell teilweise sehr aufwendige Methodik und die mögliche Kreuzreaktion mit therapeutisch nicht wirksamen Verbindungen. Chromatografische Methoden nutzen die Trennung der nachzuweisenden Verbindung von die Detektion störenden Substanzen mittels physikalischer Effekte. Die Empfindlichkeit des Nachweises wird dadurch stark durch die Güte des Detektors als auch durch die Qualität der Trennung beeinflußt. Besondere Nachteile dieses verfahrens sind die aufwendige notwendige Gerätetechnik und die relativ große Materialmenge, um beispielsweise den Cyclosporinspiegel im Blutplasma bestimmen zu können.

Ziel der Erfindung ist die Bereitstellung eines einfachen Verfahrens zur quantitativen Bestimmung von biologisch wirksamen Cyclosporinen in humanen Materialien von Patienten, die mit dieser Verbindungsklasse therapiert werden.

Aufgabe der Erfindung ist die Bereitstellung eines einfachen Verfahrens und eines Testkits zur quantitativen Bestimmung von biologisch wirksamen Cyclosporinen mit hoher Empfindlichkeit bei geringen Probenmengen in humanen Materialien von Patienten, die mit dieser Verbindungsklasse therapiert werden.

Die Aufgabe wird dadurch gelöst, daß nach Zusatz einer Peptidyl-Prolyl cis-trans Isomerase, welche die Peptidbindung eines Nterminalen Prolins isomerisiert, die Hemmung der enzymkatalysierten Isomerisierung eines prolinhaltigen Subtrates gemessen und daraus über eine Eichkurve die Konzentration von Cyclosporinen ermittelt wird.

Als Cyclosporine gelten auch die Verbindungen, die sich chemisch von der Substanzklasse der Cyclosporine im Sinne von Derivaten ableiten und biologisch wie Cyclosporin A wirksam sind. Zur Ausführung des Verfahrens wird katalytisch aktive und für Cyclosporine empfindliche Peptidyl-Prolyl cis-trans Isomerase (PPlase) eingesetzt.

Geeignete Substrate sind Verbindungen des Types Xaa-Pro-Yaa, mit deren Hilfe die Reaktion der PPlase nachgewiesen werden kann. Vorzugsweise werden dabei solche Substrate genutzt, in denen Yaa für eine chromogene Gruppierung, wie z.B. Phenyl-alanyl-4-nitroanilid, und in denen Xaa für Aminoacylreste oder Peptidylreste, wie z.B. Suc-Ala-Ala, steht. Ein weiteres Kennzeichen geeigneter Substrate ist die Substrateigenschaft für das zum Nachweis der PPlase notwendige Indikatorsystem mittels einer geeigneten Protease, wie z.B. Chymotrypsin, Thrombin oder humane Leukozyten Elastase unter Millieubedingungen

3

(pH-Wert, Pufferzusammensetzung, Temperatur und Ionenstärke), die für diese proteolytischen Systeme bekannt sind. Eine für Cyclosporine empfindliche PPlase mit einem Molekulargewicht von 17 kD kann beispielsweise aus Schweinenieren nach besten Vorschriften isoliert werden (Fischer G., Bang H., Mech C. Biomed. Biochim. Acta 43, 1101-1111 (1984) ). Die Cyclosporinkonzentration kann aus dem Vergleich der Aktivitäten von Messungen ohne Cyclosporin (Vergleichswert) und Messung der pplase-Aktivität mit Cyclosporingabe ermittelt werden, dabei können sowohl körpereigene Isomerase als auch im Verfahren enthaltene isomerisierende Aktivitäten als Maß für den Cyclosporinspiegel dienen. Die Durchführung der Aktivitätsbestimmung der PPlase einschließlich der Konzentrationsbestimmung von Cyclosporinen soll im folgenden an zwei Ausführungsbeispielen näher erläutert werden.

Ausführungsbeispiele

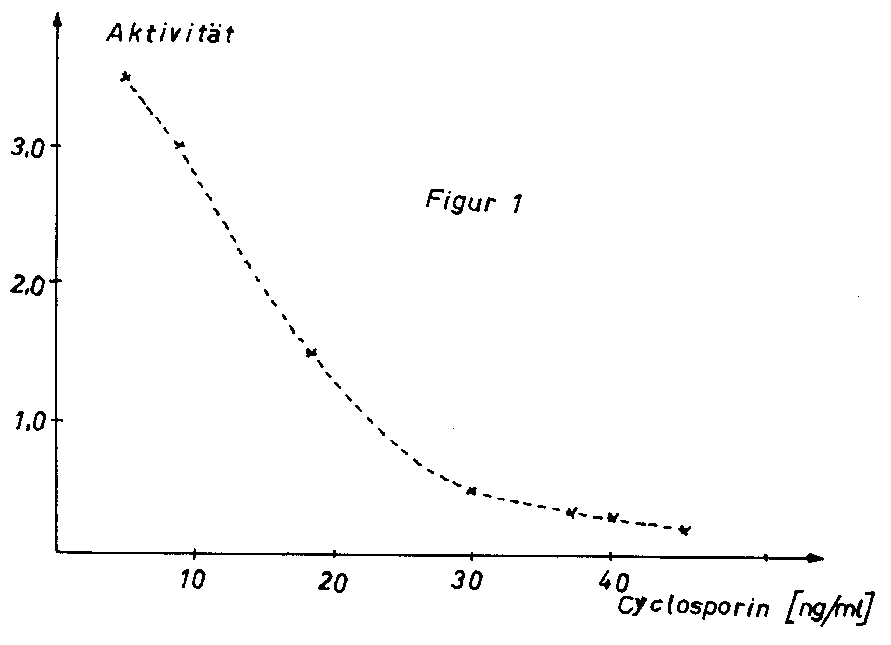

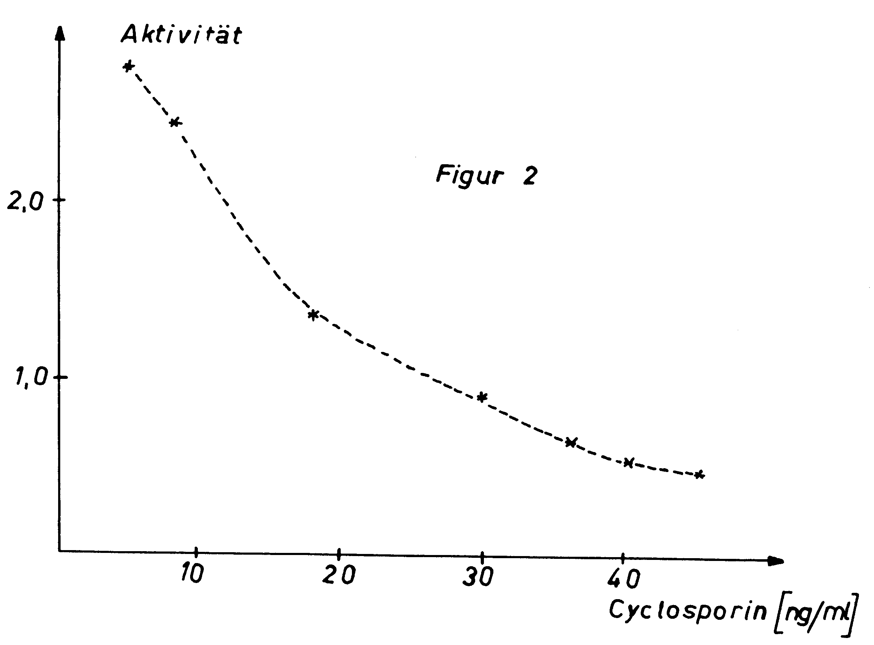

Berechnung: Üblicherweise wird die Konzentration eines Hemmstoffes aus der prozentualen Inhibierung einer bestimmten zugesetzten Enzymmenge anhand einer Eichkurve gemessen. Eine solche typische Eichkurve ist in Fig. 1 für Ausführungsbeispiel 1 und in Fig. 2 für Ausführungsbeispiel 2 dargestellt.

Beispiel 1:

Puffer: 0,035 m HEPES pH 7,8

Chymotrypsin: alpha-Chymotrypsin aus Rinderpankreas (3-fach kristallisiert, Boehringer/BRD) 24 mg in 4,2 ml Puffer

Substrat: Glt-Ala-Ala-Pro-Phe-NHNp 14 mg in 10 ml Dimethylsulfoxid

PPlase: aus Schweinenieren isoliert spez.Aktivität = 500 $\mu$Mol/min x mg

Meßgerät: registrierendes Spektrallinienphotometer

Meßwellenlänge: 410 nm

Vorinkubationszeit: 20 Minuten

Reaktionstemperatur: 5°C

Probe: humaner Plasmapool, dem 5,8,18,30,36,40 ng/ml Cyclosporin-A zugesetzt werden.

| | Meßansatz | Leeransatz |
|---|---|---|
| Puffer: | 500 $\mu$l | 540 $\mu$l |
| Chymotrypsinlösung: | 100 $\mu$l | 100 $\mu$l |
| PPlase-Lösung: | 20 $\mu$l | - |
| Probe: | 20 $\mu$l | - |
| Substratlösung: | 50 $\mu$l | 50 $\mu$l |

Aktivitätsbestimmung:

a) Berechnung der Geschwindigkeitskonstante von Meßansatz (k-Meßansatz) und Leeransatz (k-Leeransatz) der Kinetik nach einem Zeitgesetz erster Ordnung

b) Berechnung der gemessenen Aktivität der Probe nach:

$$\text{Aktivität} = \frac{\text{k-Meßansatz}}{\text{k-Leeransatz}} - 1$$

Cyclosporinkonzentration: Ermittlung mit Hilfe einer Eichkurve wie in Fig. 1 angegeben.

Beispiel 2:

Puffer: 0,035 m HEPES ph 7,8

Chymotrypsin: alpha-Chymotrypsin aus Rinderpankreas (3-fach kristallisiert, Boehringer/BRD) 35 mg in 4,2 ml Puffer

Substrat: Glt-Ala-Pro-Phe-NHNp 14 mg in 10 ml Dimethylsulfoxid

PPlase: aus Schweinenieren isoliert spez. Aktivität = 500 $\mu$Mol/min x mg

Meßgerät: registrierendes Spektrallinienphotometer
Meßwellenlänge: 410 nm
Vorinkubationszeit: 20 Minuten
Reaktionstemperatur: 5 °C
Probe: humaner Plasmapool, dem 5,8,18,30,36,40 ng/ml Cyclosporin-A zugesetzt waren
Meßansatz, Leeransatz und Aktivitätsbestimmung wie in Beispiel 1
Cyclosporinkonzentration: Ermittlung mit Hilfe einer Eichkurve entsprechend Fig.2

## Patentansprüche

1. Verfahren zur Konzentrationsbestimmung von Cyclosporinen, dadurch gekennzeichnet, daß nach Zusatz einer Peptidyl-Prolyl cis-trans Isomerase, welche die Peptidbindung eines N-terminalen Prolins isomerisiert, die Hemmung der enzymkatalysierten Isomerisierung eines prolinhaltigen Substrates gemessen und daraus über eine Eichkurve die Konzentration von Cyclosporinen ermittelt wird.

2. Verfahren gemäß Anspruch 1, dadurch gekennzeichnet, daß als prolinhaltige Substrate Verbindungen des Typs Xaa-Pro-Yaa eingesetzt werden, in denen Yaa für eine chromogene Gruppierung, z.B. Phenylalanyl-4-nitroanilid oder Alanyl-4-nitroanilid und Xaa für Aminoacyl- oder Peptidylreste, z.B. Suc-Ala-Ala, steht.

3. Verfahren gemäß den Ansprüchen 1 und 2, dadurch gekennzeichnet, daß die Isomerasesubstrate auch Substrateigenschaft für das zum Nachweis der Hemmung der Isomerase notwendige Indikatorsystem mit einer Protease, z.B. Chymotrypsin, Thrombin oder humane LeukozytenElastase aufweist.

4. Testkit zur Konzentrationsbestimmung von Cyclosporinen nach den Ansprüchen 1 bis 3, gekennzeichnet durch folgende Zusammensetzung:
   - Peptidiyl-Prolyl-cis-trans-Isomerase MG 17 KD,
   - ein Hilfsstoff mit Proteaseeigenschaften, wie Chymotrypsin, Thrombin oder humane Leukozytenelastase,
   - ein Substrat des Typs Xaa-Pro-Yaa, worin Yaa für eine chromogene Gruppierung und Xaa für Aminoacyl oder Peptidylreste, wie Suc-Ala-Ala, stehen und Puffer, vorzugsweise HEPES-Puffer vom pH 7,8.

5. Testkit nach Anspruch 4, dadurch gekennzeichnet, daß Yaa für Phenylalanyl-4-nitroanilid oder Alanyl-4-nitroanilid steht.

## Claims

1. A method of determining the concentration of cyclosporins, characterized in that, after the addition of a peptidylprolyl cis-trans isomerase, which isomerizes the peptide bond of an N-terminal proline, the inhibition of the enzyme-catalyzed isomerization of a proline-containing substrate is measured and the concentration of cyclosporins is determined therefrom *via* a calibration curve.

2. A method according to Claim 1, characterized in that the proline-containing substrates used are compounds of the type Xaa-Pro-Yaa, in which Yaa is a chromogenic grouping, *e.g.* phenylalanyl-4-nitroanilide or alanyl-4-nitroanilide, and Xaa is aminoacyl or peptidyl residues, *e.g.* Suc-Ala-Ala.

3. A method according to Claims 1 and 2, characterized in that the isomerase substrates also have the property of substrates for the indicator system required to detect the inhibition of the isomerase, with a protease, *e.g.* chymotrypsin, thrombin or human leukocyte elastase.

4. A test kit for determining the concentration of cyclosporins according to Claims 1 to 3, characterized in that it has the following composition:
   - peptidyl-prolyl cis-trans isomerase of MW 17 kD,
   - an adjunct with protease properties, such as chymo trypsin, thrombin or human leukocyte elastase,
   - a substrate of the type Xaa-Pro-Yaa, in which Yaa is a chromogenic grouping and Xaa is aminoacyl or peptidyl residues such as Suc-Ala-Ala, and a buffer, preferably HEPES buffer of pH

EP 0 360 029 B1

7.8.

**5.** A test kit according to Claim 4, characterized in that Yaa is phenylalanyl-4-nitroanilide or alanyl-4-nitroanilide.

**Revendications**

**1.** Procédé pour la détermination de la concentration en cyclosporines, caractérisé en ce qu'après addition d'une peptidyl-prolyl cis-trans isomérase qui isomérise la liaison peptide d'une proline N-terminale, on mesure l'inhibition de l'isomérisation catalysée par enzyme d'un substrat contenant la proline et à partir de là on détermine par une courbe d'étalonnage la concentration de cyclosporines.

**2.** Procédé selon la revendication 1, caractérisé en ce qu'en tant que substrats à teneur de proline, on met en oeuvre des composés du type Xaa-Pro-Yaa, dans lesquels Yaa signifie un groupement chromogène par exemple phénylalanyl-4-nitroanilide ou alanyl-4-nitroanilide et Xaa signifie des restes aminoacyle ou peptidyle, par exemple Suc-Ala-Ala.

**3.** Procédé selon les revendications 1 et 2, caractérisé en ce que le substrat d'isomérase présente également des propriétés de substrat pour le système indicateur pour démontrer l'inhibition de l'isomérase avec une protéase, par exemple la chymotrypsine, la thrombine ou l'élastase leucocytaire humaine.

**4.** Nécessaire d'essai ou kit pour la détermination de la concentration en cyclosporines selon les revendications 1 à 3, caractérisé par la composition suivante :
  - peptidiyl-prolyl cis-trans isomérase MG 17 KD,
  - un adjuvant avec des propriétés de protéase tel que la chymotrypsine, la thrombine ou l'élastase leucocytaire humaine,
  - un substrat du type Xaa-Pro-Yaa, où Yaa signifie un groupement chromogène et Xaa des restes aminoacyle ou peptidyle, tel que Suc-Ala-Ala et un tampon, de préférence un tampon HEPES de pH 7,8.

**5.** Nécessaire d'essai ou kit selon la revendication 4, caractérisé en ce que Yaa signifie phénylalanyl-4-nitroanilide ou alanyl-4-nitroanilide.

6

Figur 1

EP 0 360 029 B1

Figur 2

Aktivität

Cyclosporin [ng/ml]